**Europäisches Patentamt**

**European Patent Office**

**Office européen des brevets**

(19)

(11) Veröffentlichungsnummer: **0 036 530**
A2

(12) **EUROPÄISCHE PATENTANMELDUNG**

(21) Anmeldenummer: **81101594.0**

(22) Anmeldetag: **05.03.81**

(51) Int. Cl.³: **G 01 T 1/20**

(30) Priorität: **13.03.80 DE 3009723**

(43) Veröffentlichungstag der Anmeldung: **30.09.81**
**Patentblatt 81/39**

(84) Benannte Vertragsstaaten: **DE FR GB**

(71) Anmelder: **SIEMENS AKTIENGESELLSCHAFT Berlin und München, Postfach 22 02 61, D-8000 München 22 (DE)**

(72) Erfinder: **Grassmann, Peter, Dr.-Ing., Schronfeld 12, D-8520 Erlangen (DE)**
Erfinder: **Pfeiler, Manfred, Dr.-Ing., Ludwig-Thoma-Strasse 25, D-8520 Erlangen (DE)**

(54) **Strahlenmesseinrichtung.**

(57) Die Erfindung bezieht sich auf eine Strahlenmeßeinrichtung mit einer Strahlenquelle (1), die ein fächerförmiges Strahlenbündel (4) aussendet und einem Strahlenempfänger (2), der eine Reihe lichtelektrischer Wandler (2a, 2b usw.) aufweist, wobei vor jedem der lichtelektrischen Wandler ein Szintillationskristall (3) angeordnet ist. Es ist ein einziger, langgestreckter Szintillationskristall (3) für mehrere Wandler (2a, 2b usw.) vorhanden, mit dem die zugeordneten Wandler (2a, 2b usw.) verbunden sind.

SIEMENS AKTIENGESELLSCHAFT
Berlin und München

0036530

Unser Zeichen
VPA 80 P 5031 E

## Strahlenmeßeinrichtung

Die Erfindung betrifft eine Strahlenmeßeinrichtung mit einer Strahlenquelle, die ein fächerförmiges Strahlenbündel aussendet und einem Strahlenempfänger, der eine Reihe von lichtelektrischen Wandlern aufweist, vor denen ein Szintillationskristall angeordnet ist.

Eine Strahlenmeßeinrichtung dieser Art wird in einem Computertomographen benutzt und dient zur Abtastung einer Transversalschicht eines Aufnahmeobjektes unter verschiedenen Projektionen. Ein Rechner bestimmt aus den Ausgangssignalen der Wandler bei den verschiedenen Projektionen die Schwächungskoeffizienten von in einer Matrix angeordneten Bildpunkten. Die berechneten Schwächungskoeffizienten können dann als Bild der abgetasteten Transversalschicht auf einem Sichtgerät wiedergegeben werden.

Bei einer bekannten Strahlenmeßeinrichtung der eingangs genannten Art wird jeder als Fotohalbleiter ausgebildete Wandler mit je einem Szintillationskristall kombiniert. Dies bedeutet jedoch häufig einen großen Aufwand, denn bei einem Computertomographen werden zur Erzielung einer ausreichenden Bildauflösung sehr viele Wandler, beispielsweise 512 Wandler, verwendet.

Der Erfindung liegt die Aufgabe zugrunde, eine Strahlenmeßeinrichtung der eingangs genannten Art zu schaffen, die einfach aufgebaut und herstellbar ist.

Tp 5 Kli / 25.2.1980

Diese Aufgabe ist erfindungsgemäß dadurch gelöst, daß ein einziger, langgestreckter Szintillationskristall vorhanden ist, mit dem die zugeordneten Wandler verbunden sind. Bei der erfindungsgemäßen Strahlenmeßeinrichtung ist nicht für jeden Wandler je ein gesonderter Szintillationskristall vorhanden, so daß die Herstellung des gesamten Strahlenempfängers vereinfacht und verbilligt ist.

Einzelheiten der Erfindung ergeben sich aus den Unteransprüchen.

Die Erfindung ist nachfolgend anhand eines in der Zeichnung dargestellten Ausführungsbeispiels näher erläutert. Es zeigen:

Fig. 1 eine Darstellung einer Strahlenmeßeinrichtung, bei der der Erfindungsgedanke angewandt ist und

Fig. 2 bis 5 verschiedene Ausführungen des Strahlenempfängers einer Strahlenmeßeinrichtung nach der Erfindung.

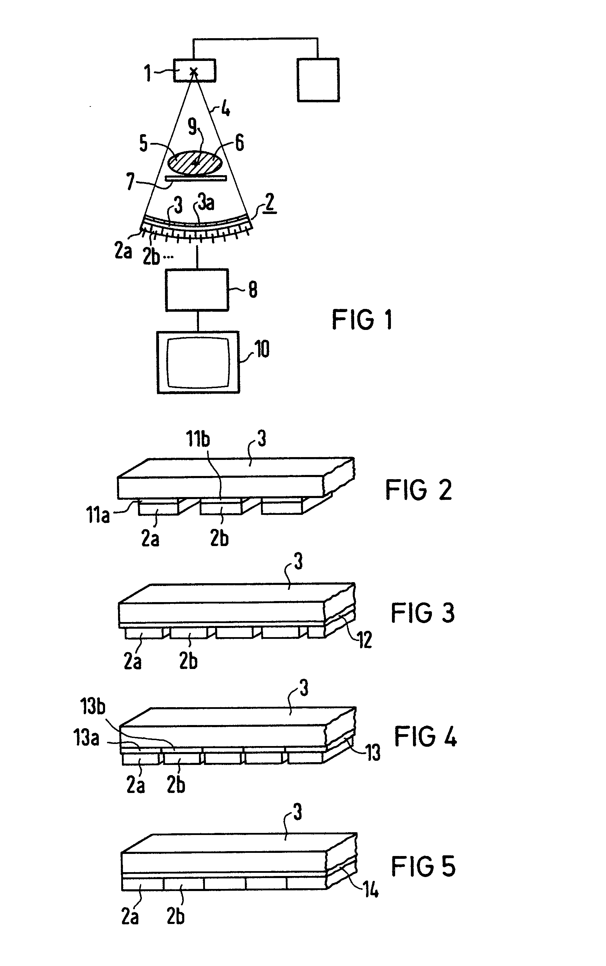

In der Figur 1 ist als Strahlenquelle eine Röntgenröhre 1 dargestellt, die mit einem Strahlenempfänger 2 eine Strahlenmeßeinrichtung bildet. Der Strahlenempfänger 2 weist eine Reihe von lichtelektrischen Wandlern 2a, 2b usw. aus Halbleitermaterial auf, vor denen ein einziger Szintillationskristall 3 und ein Kollimator 3a, dessen Lamellen auf den Fokus der Röntgenröhre 1 ausgerichtet sind, angeordnet sind. Die Röntgenröhre 1 ist mit dem Strahlenempfänger 2 über einen Drehrahmen fest verbunden. Die Röntgenröhre 1 sendet ein fächerförmiges Röntgenstrahlenbündel 4 aus, dessen Ausdehnung in der zu untersuchenden Querschicht 5 eines Patienten 6, welcher auf einer Lagerstatt 7 liegt, so groß ist, daß die gesamte zu untersuchende Schicht 5 von Röntgenstrahlung durchsetzt wird. Senkrecht zu der Schicht 5 entspricht die Ausdehnung des Röntgenstrahlenbündels 4 der Schichtstärke. Die Anzahl der Wandler des Strahlenempfängers 2 ist der gewünschten Bildauflösung entsprechend gewählt und kann z.B. 512 betragen. Jeder Wandler liefert ein Signal, das der Intensität der empfangenen Röntgenstrahlung entspricht.

Die Wandler 2a, 2b usw. des Strahlenempfängers 2 sind an einem Rechner 8 angeschlossen, der aus den Ausgangssignalen der Detektoren, die während der Drehung der Meßanordnung 1, 2 um einen Winkel von $360^{o}$ um die Drehachse 9 gebildet werden, die Schwächungswerte bestimmter Bildpunkte der Schicht 5 und damit ein Bild der durchstrahlten Schicht 5 des Patienten 6 berechnet. Dieses Bild wird auf einem Sichtgerät 10 wiedergegeben.

Zur Verringerung der Strahlenbelastung des Patienten 6 kann die Röntgenröhre 1 während eines Abtastvorganges gepulst werden, so daß beispielsweise pro Winkelgrad ein Satz von Ausgangssignalen des Strahlenempfängers 2 erzeugt wird. Auf diese Weise werden beispielsweise 360 x 512 Ausgangssignale erzeugt. Bei dem Beispiel sind der Übersichtlichkeit halber nicht alle Wandler, sondern nur eine geringe Anzahl gezeigt.

Wesentlich ist, daß für alle Wandler 2a, 2b usw. ein einziger, langgestreckter Szintillationskristall 3 vorhanden ist, mit dem alle Wandler verbunden sind.

Aus der Fig. 2 geht hervor, daß die Wandler 2a, 2b usw. einzeln am Szintillationskristall 3 mit Hilfe von Klebeschichten 11a, 11b usw. befestigt sind.

Die Fig. 3 zeigt ein Ausführungsbeispiel, bei dem der Szintillationskristall 3 mit einer durchgehenden Klebeschicht 12 versehen ist, auf die die Wandler 2a, 2b usw. aufgeklebt sind.

Aus der Fig. 4 geht hervor, daß eine durchgehende Klebeschicht 13 vorhanden ist, mit der wie bei dem Beispiel gemäß Fig. 3 die Wandler 2a, 2b usw. verbunden sind, die aber in einzelne, lichtdurchlässige Abschnitte 13a, 13b usw. unterteilt ist.

Schließlich zeigt die Fig. 5 ein Beispiel, bei dem die Wandler 2a, 2b usw. des Strahlenempfängers eine zusammenhängende Reihe bilden, die mit Hilfe einer Klebeschicht 14 mit dem Szintillationskristall 3 verbunden ist. Auch hier kann die Klebeschicht 14 aus einzelnen, lichtdurchlässigen Abschnitten bestehen.

Der Szintillationskristall 3 kann als auf den Detektoren aufgedampfte Schicht ausgebildet werden. Ein solches Aufdampfen ist insbesondere dann sinnvoll, wenn die Detektoren eine zusammenhängende Reihe bilden.

Der Strahlenempfänger 2 kann auch aus mehreren Komponenten der beschriebenen Art, von denen jede aus einem einzigen Szintillationskristall und mehreren damit verbundenen lichtelektrischen Wandlern besteht, aufgebaut sein. Er kann geradlinig oder um den Fokus der Strahlenquelle gekrümmt sein.

5 Figuren
6 Patentansprüche

0036530

<u>Patentansprüche</u>

1. Strahlenmeßeinrichtung mit einer Strahlenquelle, die ein fächerförmiges Strahlenbündel aussendet und einem Strahlenempfänger, der eine Reihe von lichtelektrischen Wandlern aufweist, vor denen ein Szintillationskristall angeordnet ist, d a d u r c h   g e k e n n z e i c h - n e t , daß ein einziger, langgestreckter Szintillationskristall (3) für mehrere Wandler (2a, 2b usw.) vorhanden ist, mit dem die zugeordneten Wandler (2a, 2b usw.) verbunden sind.

2. Strahlenmeßeinrichtung nach Anspruch 1, d a - d u r c h   g e k e n n z e i c h n e t , daß die. Wandler (2a, 2b usw.) einzeln am Szintillationskristall (3) angeklebt sind.

3. Strahlenmeßeinrichtung nach Anspruch 1, d a - d u r c h   g e k e n n z e i c h n e t , daß der Szintillationskristall (3) mit einer durchgehenden Klebeschicht (12, 13, 14) versehen ist, auf die die Wandler (2a, 2b usw.) aufgeklebt sind.

4. Strahlenmeßeinrichtung nach Anspruch 3, d a - d u r c h   g e k e n n z e i c h n e t , daß die Klebeschicht (13) aus den Wandlern (2a, 2b usw.) gegenüberliegenden lichtdurchlässigen Abschnitten besteht.

5. Strahlenmeßeinrichtung nach einem der Ansprüche 1, 3 oder 4, d a d u r c h   g e k e n n z e i c h n e t, daß die Wandler (2a, 2b usw.) eine zusammenhängende Reihe bilden.

6. Strahlenmeßeinrichtung nach einem der Ansprüche 1 bis 5, d a d u r c h   g e k e n n z e i c h n e t , daß der Szintillationskristall (3) auf den Wandlern (2a, 2b usw.) aufgedampft ist.

FIG 1

FIG 2

FIG 3

FIG 4

FIG 5